# EUROPEAN PATENT SPECIFICATION

(11) **EP 0 792 354 B1**
(45) Date of publication and mention of the grant of the patent: **22.01.2003**
(21) Application number: 95937931.4
(22) Date of filing: 16.11.1995
(51) Int. Cl.: C12N 15/10, C12N 5/10, C12N 15/63, C12N 15/85, A61K 35/14, A61K 39/00, C07K 14/725

(54) **TARGETED T LYMPHOCYTES**
GEZIELTE T-LYMFOZYTEN
LYMPHOCYTES T CIBLES

(30) Priority: 16.11.1994 GB 9423085
(43) Date of publication of application: 03.09.1997
(73) Proprietor: CELLFACTORS PLC, Cambridge CB4 0WS (GB)
(72) Inventor: Stringer, Bradley Michael John, Cycoed, Cardiff CF2 6BS (GB)
(74) Representative: Davies, Jonathan Mark
(86) International application number: GB9502691
(87) International publication number: WO96015238

(56) References cited:
- EP-A- 0 180 878
- WO-A-91/01133
- WO-A-92/12996
- NATURE, vol. 320, 20 March 1986 LONDON, GB, pages 232-238, Z. DEMBIC ET AL. 'Transfer of specificity by murine alpha and beta T-cell receptor genes.' cited in the application
- PROCEEDINGS OF THE NATIONAL ACADEMY OF SCIENCES OF THE USA, vol. 86, no. 24, December 1989 WASHINGTON, DC, USA, pages 10024-10028, G. GROSS ET AL. 'Expression of immunoglobulin-T-cell receptor chimeric molecules as functional receptors with antibody-type specificity.'
- PROCEEDINGS OF THE NATIONAL ACADEMY OF SCIENCES OF THE USA, vol. 84, May 1987 WASHINGTON, DC, USA, pages 2936-2940, N. GASCOIGNE ET AL. 'Secretion of a chimeric T-cell receptor-immunoglobulin protein.'

## Description

The present invention relates to targeted cytotoxic T lymphocytes and their uses, and in particular to targeted cytotoxic T lymphocytes having heterologous α and β T-cell antigen receptor polypeptides which confer MHC-restricted specificity for disease-causing target cells.

The immune system comprises a multitude of different cell types and molecules distributed throughout the body. In healthy individuals, the immune system defends and protects against invading pathogens, parasites and cells which have become infected or cancerous.

The cellular basis for several fundamental properties of the immune system (including immunological memory, specificity and diversity of responses) is the lymphocyte. Lymphocytes fall into two classes: the B-lymphocyte (or B-cell), which is ultimately responsible for generating humoral responses (mediated by soluble antibodies) and the T-lymphocyte (or T-cell), which is ultimately responsible for generating cell-mediated responses.

T-lymphocytes are further divided into inter alia cytotoxic T lymphocytes (which identify and kill infected, aberrant or malignant target cells) and helper T lymphocytes (which stimulate, recruit and activate a broad range of cell-types involved in mounting an immune response).

T-lymphocytes recognize target cells via T cell receptors (hereinafter, TcRs). TcRs do not generally show binding to antigen alone. Rather, TcRs recognize antigen as part of a cell-surface glycoprotein complex encoded by class I or class II genes of the major histocompatibility complex (hereinafter referred to as the MHC Class I or MHC Class II). Cytotoxic T lymphocytes generally recognize antigens in association with MHC Class I, whereas helper T lymphocytes generally recognize antigens in association with MHC Class II.

As a result, T-lymphocyte target cell recognition is restricted to target cells capable of presenting antigen in association with particular host proteins (viz. the class I or class II MHC molecules). The primary role of the T-lymphocyte may therefore be viewed as the monitoring of cell surfaces in the body so that aberrant, infected or malignant cells can be identified (or detected, in the case of helper T cells) and ultimately eliminated.

### The T cell Receptor (TcR)

The TcR is a protein complex, one type of which includes two disulphide cross-linked polypeptide chains (the α and β chains) which are associated on the T lymphocyte cell surface with other molecules. The α and β chains of the TcR are sufficient to endow a T lymphocyte with both antigen and MHC (of either Class I or II) specificity: only the α and β components of the TcR are therefore required to define completely the dual (i.e. MHC and antigen) T lymphocyte specificity.

The α and β chains comprise constant and variable regions which are homologous to immunoglobulin V and C regions, and the corresponding structural genes undergo DNA arrangements reminiscent of those of the immunoglobulin genes. Indeed, the structural repertoire of immunoglobulins and TcRs are similar (about 10¹² per individual).

The genes encoding the α and β chains of the TcR have been cloned and sequenced (see e.g. Robertson (1985), Nature 317, 768-771). Moreover, TcR α and β-chain genes have been transferred from one T cell to another to generate a T cell clone of different specificity (see e.g. Dembic et al., 1986, Nature 320, pp. 232-238).

TcRs are clonally distributed: the TcRs of each T lymphocyte are specific for just one antigenic determinant (though there may be hundreds of thousands of the same species of TcR on each cell). Thus, each T lymphocyte is normally monovalent with respect to TcR-mediated binding.

The α and β chains are associated with several other molecules, including proteins of the CD3 complex and associated zeta and eta peptides. The TcR-CD3 complex transduces a signal from the TcR to the interior of the T lymphocyte when it recognizes the appropriate peptide-MHC complex, thus contributing to T-cell activation.

### Aberrant gene expression, disease and the T cell response

The health of an individual depends on the co-ordinated and tightly-regulated expression of many thousands of different genes. Aberration or alteration of gene expression, even if subtle, can therefore give rise to a plethora of different diseases, including cancers, AIDS and various autoimmune diseases.

Many different factors may induce aberrant gene expression. For example, the expression of endogenous genes or the structure of the proteins encoded thereby may be altered by mutation. Such mutations may be spontaneous, but are often induced by carcinogens present in the environment. Alternatively, the introduction of exogenous genetic material into cells of the individual (for example as a result of viral infection) may result in the disruption of gene expression patterns, the activation of normally silent genes and/or the synthesis of foreign (e.g. viral) proteins.

One important example of the consequences of induced aberrations in gene expression is that of tumour formation. Tumour formation characterizes most cancers, and the tumours arise as a consequence of the structural alteration or overexpression of endogenous proteins attendant on the mutation of oncogenes and tumour suppressor genes in somatic cells. For example, in certain types of breast cancer tumour formation arises from amplification and overexpression of the HER-2/neu protooncogene.

Apart from the onset of disease at the organismal level, another phenotypic consequence of aberrant gene expression may be the production of neoantigens. As used herein, the term "neoantigen" is intended to cover not only new antigens arising from mutant or foreign amino acid sequences, but also "new" antigens arising from the expression of normally silent wild-type genes, or the overexpression of normally relatively weakly-expressed wild-type genes.

The production of neoantigens may give rise to alterations in cell surface antigens, which alterations can in turn induce an immune response. For example, antibodies directed against oncogene products have been found in the serum of tumour patients and cytotoxic T lymphocytes which recognize and eliminate tumour cells have been demonstrated in a number of model systems.

Alterations in cell surface antigens capable of triggering a cytotoxic T lymphocyte response may arise following intracellular processing of the mutant, foreign or overexpressed proteins to produce peptide fragments which may be displayed at the cell-surface as part of a peptide-MHC Class I complex capable of recognition by cytotoxic T lymphocytes. In this way, aberrant cells may be recognized and eliminated by T lymphocytes.

In addition to alterations in cell surface antigens, protein overexpression, foreign (e.g. viral-derived) or mutant protein production may ultimately result in the appearance of exogenous neoantigens (e.g. virus particles or fragments). These may be recognized by membrane-bound immunoglobulins on the surface of B-lymphocytes, internalized, processed and subsequently presented as helper T-cell antigens as a complex with MHC class II molecules at the surface of the B-cell. A B-cell which is presenting antigen in this way can be recognized by a helper T-cell via a specific TcR-peptide-MHC Class II interaction and stimulated to develop into a plasma cell secreting large quantities of soluble antibodies specific for the exogenous antigen. In this way the helper T lymphocyte may stimulate a humoral immune response and indirectly activate a wide range of immune cell-types.

There are many reports of tumour-specific or tumour-associated neoantigens. For example, overexpression of the ERBB2 receptor is associated with many human breast and ovarian cancers and also results in the appearance of ERBB2-derived neoantigens at the cell surface. In colon cancer, the adenomatosis polyposis coli gene (APC) usually undergoes early mutation. Although the mutation often simply results in the introduction of a stop codon (which terminates translation resulting in the production of truncated APC protein), in many cases the mutation is a frameshift which results in the production of new protein sequences and potentially unique antigens. Once processed intracellularly and bound to the MHC proteins, such antigens may be presented at the cell surface and recognized by T lymphocytes.

In many other cases the structural alteration or overexpression of endogenous proteins would be expected to result in alterations in the intracellular processing of these proteins and the presentation of neoantigens in association with MHC proteins on the cell surface.

Despite the above-described immunogenic consequences of aberrant gene expression, the natural immune response mounted to combat infected, aberrant or malignant cells is often inadequate. Although the reasons for this failure are not fully understood, it is thought that neoantigens are often treated by the immune system as tissue-specific antigens and so tolerated in the same way. Moreover, neoantigens usually constitute only a minor source of peptides for MHC-class I directed processing, and so neoantigen processing and presentation may be extremely inefficient. Finally, immunogenicity appears to be determined in part by the tissue type in which the neoantigen is expressed, and so it seems that tolerance may be greater (and/or processing and presentation less efficient) in certain tissue types.

Several approaches have been developed to generate more effective immune responses against aberrant, infected or malignant cellular targets. For example, it has been proposed to use antigens from malignant cells as vaccines to induce tumour-specific cell-mediated immunity.

More recently, adoptive immunotherapy has been proposed as a mode of cancer treatment. This mode of therapy is based on the transfer of immune cells with antitumour activity into cancer patients. The immune cells used are derived from the cancer patient, cultured, and reintroduced after expansion in tissue culture.

The cells used in adoptive immunotherapy include lymphokine-activated killer cells, tumour-infiltrating lymphocytes and in vitro sensitized lymphocytes derived from cytotoxic T lymphocytes.

Lymphokine-activated killer cells are cytolytic cells which react with a broad spectrum of target cells. They are not MHC-restricted and lyse both tumour and normal cells. Therapies based on the use of these cells therefore suffer the disadvantage that tumour cell killing is accompanied with significant damage to normal tissue.

Tumour-infiltrating lymphocytes are derived from tumour tissues. They are more potent than lymphokine-activated killer cells and relatively specific for their tumours of origin, thus avoiding the problems arising from non-selective ablation of normal tissue associated with the use of lymphokine-activated killer cells. However, the availability of these cells is severely restricted, being dependent on their natural occurrence in tumour tissues and their expansion in vitro. Thus, therapies based on the use of these cells would be available in only a fraction of cases.

While each of the adoptive immunotherapies discussed above have been shown to be capable of mediating tumour regression to some extent, therapeutic responses have been observed in only a few cases (and even then were exhibited in only a fraction of the patients treated).

It has been proposed (Moritz et al. (1994), PNAS, 91, pp. 4318-4322) to improve the efficacy of lymphocyte-mediated tumour therapy by in vitro manipulation of the recognition specificity of cytotoxic T lymphocytes to endow them with a defined tumour cell specificity.
Moritz et al. approached this problem by circumventing the TcR-based (and therefore MHC-restricted) specificity to create a wholly synthetic "pseudoreceptor" comprising a single chain antibody (to confer binding specificity) joined (via a hinge) to a TcR-complex zeta chain. Cytotoxic T cells bearing the synthetic pseudoreceptor were found to exhibit MHC-independent recognition with a specificity conferred by the antibody component.

However, the use of a wholly synthetic pseudoreceptor appears to lead to inefficient signal transduction and a reduction in T cell activity. Moreover, since the recognition specificity of the targeted cytotoxic T lymphocytes described in Moritz et al. is MHC-independent, the efficiency of cell-surface scanning by the lymphocytes is likely to be impaired. Finally, the synthetic pseudoreceptor might itself elicit undesirable immune responses.

It is an object of the present invention to provide alternative targeted T lymphocytes for use e.g. in adoptive immunotherapy which do not express wholly synthetic pseudoreceptors, but are instead targeted via TcRs which are essentially similar in structure to normal TcRs. The normal signal transduction pathway is therefore preserved and the risks of undesirable immune responses minimized. Moreover, the targeted T cells of the invention are MEC-restricted so that they can efficiently scan cell surfaces for aberrant antigens.

Accordingly, the present invention provides a targeted cytotoxic T lymphocyte having a TcR comprising heterologous α and β polypeptides, which heterologous polypeptides confer on the lymphocyte MEC Class I-restricted specificity for disease-causing target cells, wherein the targeted cytotoxic T lymphocyte is monovalent with respect to the targeted cells, having a single species of TcR conferring MHC Class I-restricted specificity for a single class of target cells.

As used herein the term "heterologous α and β TcR polypeptides" is intended to denote α and β TcR chains which are not normally expressed in the T-cell. In one embodiment, the heterologous TcR polypeptides are chimaeras or synthetic molecules derived from the expression of recombinant or wholly synthetic DNA. In other embodiments the heterologous TcR polypeptides are derived, directly or indirectly (e.g. via a TcR gene library), from another T-cell. The other T-cell may itself be derived from any source, so long as it expresses TcR components of the required specificity. For example, the other T-cell may be from the same species (or even the same individual), or from a different species. It may also be a T-cell hybridoma.

The specificity for disease-causing target cells need not be absolute. For the purposes of the invention, it is sufficient if the specificity is such that any attendant autoimmune-type ablation of normal or non-diseased tissue can be tolerated by the patient.

For example, in circumstances where the disease-causing cells are all members of a particular tissue which is itself dispensable, it is sufficient if the targeted T lymphocyte is effectively specific for that tissue. This might be the case where the targeted disease-causing cells are malignant prostate tumour cells - here it is sufficient if the cytotoxic T-cells are targeted with sufficient specificity to ablate both normal and cancerous prostate tissue (while other tissues are unaffected or ablated to a lesser and tolerable extent). Another example is melanoma - although some melanocyte-specific antigens also appear in certain cells of the retina, brain and inner ear, these latter cells appear to be less sensitive to immunotherapy based on the melanocyte-specific antigen.

Another situation where absolute specificity may not be required arises where the normal tissue counterpart of a particular tumour express MHC Class I at extremely low levels, thereby shielding it from cytotoxic T-cell attack.

The term "disease-causing cell" is used herein in a broad sense to denote not only cells which are directly involved in disease (for example tumour cells or autoimmune cells) but also cells which are associated with the progression of disease or which help promote or maintain the disease state (for example virally-infected cells). It therefore covers any cell which is aberrant and deleterious to the health of the individual in any way.

The targeted cytotoxic T lymphocyte of the invention is preferably recombinant, being for example transduced with a viral vector.

Monovalent lymphocytes of the invention may be generated e.g. by replacing (rather than supplementing) the resident (or endocenous) α and β chain genes with heterologous α and β chains conferring the required specificity.

Monovalent lymphocytes of the invention may also be generated by downregulating or blocking the expression of the endogenous TcR by any of the wide range of techniques available. For example, targeted insertional mutagenesis may be used to disrupt the endogenous TcR genes.

The use of moncvalent lymphocytes avoids problems arising from the formation of mismatched (and therefore inactive and/or non-selective) α and β dimers. The formation of such inactive heterodimers reduces the effective cell surface density of the TcRs which may impair or prevent target cell recognition and lysis. By ensuring monovalency of the lymphocytes of the invention, the cell surface density of the TcRs is optimized.

Dembic, Nature, vol. 320, 1986 pages 232-238, discloses the transfer of alpha- and beta-chain genes from a class I MHC-restricted cytotoxic T-cell clone, after cloning into a cosmid vector, to another cytolytic T-cell clone of a different specificity by protoplast fusion. A neomycin-resistance gene was used a positive selection marker. This shows that TcR alpha- and beta-chain genes, isolated from a cytotoxic T cell, are sufficient to transfer that cell's MHC-restricted antigen specificity to another T cell.

Dembic does not teach the use of monovalent targeted cells.

Gross, Proc. Natl. Acad. Sci. USA, vol. 86, no. 24, 1989, pages 10024-10028, teaches the generation of chimeric molecules consisting of immunoglobulin V regions for antigen recognition and TcR C regions. The chimeric genes were cloned into virus-derived expression vectors and cytotoxic T-cell hybridomas were transected using the protoplast fusion technique. Expression of these chimeric TcR endowed the recipient T cell with antibody-type specificity and recognition of antigens in a non-MHC-restricted manner was possible.

However, neither Dembic nor Gross mentions the problems which may arise by the formation of mismatched dimers of TcR which results in inactive heterodimers.

The target cells preferably comprise tumour cells, immune cells contributing to an autoimmune response and/or cells infected with a pathogen (and in particular, virus-infected cells). In a particularly preferred embodiment the target cells comprise HIV-infected lymphocytes. Other viral infections include hepatitis (e.g. hepatitis B,C and NANB) and virally-induced Burkitt's lymphoma

The heterologous α and β TcR polypeptides may be chimaeric, and may for example comprise an immunoglobulin variable domain or fragment thereof. Such chimaeric TcRs exploit the close structural similarities between the constant and variable regions of the α and β chains of the TcR and immunoglobulin V and C regions. The use of such chimaeric α and β TcR polypeptides may be particularly convenient where an immunoglobulin exhibiting the required specificity is available or readily obtainable (for example by raising antibodies to known or predicted T-cell antigens).

In another aspect, the invention also contemplates a method for producing the targeted cytotoxic T lymphocyte of the invention, the method comprising the steps of: (a) providing a vector comprising DNA (e.g. DNA derived from a donor cytotoxic T lymphocyte) encoding α and β TcR polypeptides specific for disease-causing target cells, and (b) transfecting a recipient cytotoxic T lymphocyte with the vector of step (a) to produce a recombinant cytotoxic T lymphocyte having DNA encoding α and β TcR polypeptides specific for disease-causing target cells, whereby the recombinant cytotoxic T lymphocyte of step (b) expresses the DNA encoding α and β TcR polypeptides to endow the lymphocyte with MHC-Class I restricted specificity and thereby target it to the target cells.

The vector of step (a) may be provided by cloning, assembling or synthesising (e.g. by solid phase oligonucleotide synthesis) DNA encoding α and β TcR polypeptides specific for disease-causing target cells. Preferably, the DNA encoding the α and β TcR polypeptides is cloned by: (a) obtaining a sample of donor T lymphocytes, e.g. from a blood bank, blood sample or tumour biopsy; (b) enriching the sample of donor T lymphocytes for cytotoxic T lymphocytes having specificity for disease-causing target cells, e.g. by specifically induced proliferation and/or specific clonal expansion; (c) extracting chromosomal DNA from the donor cytotoxic T lymphocytes; and (d) isolating DNA encoding the α and β TcR polypeptides, e.g. by primer-specific PCR amplification.

The vector may be introduced into the recipient cytotoxic T lymphocyte by any suitable method. Many different methods are known to those skilled in the art, including transfection by electroporation, protoplast fusion or viral (e.g. retroviral) transfection.

The invention also relates in a further aspect to a vector for use in the method of the invention, which vector comprises DNA encoding α and β TcR polypeptides specific for a disease-causing cell. The DNA of the vector may preferably be operably linked to an expression element or elements to provide for expression of the TcR polypeptides at suitable levels. Any of a wide variety of expression elements may be used and the element(s) may take any form so long as they can (under at least some circumstances) be made to direct and/or control the expression of the genes with which they are operably coupled. The expression element or elements may for example comprise transcriptional and/or translational elements, and include promoters, ribosome binding sites, enhancers and regulatory sites including activator and repressor (operator) sites. By way of example only, the expression elements for use in the invention may be selected from those naturally associated with the α and/or β TcR peptide genes.

Conveniently, the vector of the invention is a viral vector, being for example based on simian virus 40, adenoviruses (e.g. human adenoviruses), retroviruses, and papillomavirus.

The vector may further comprise; (a) a positive selectable marker, the marker for example being selected from neomycin phosphotransferase, hygromycin phosphotransferase, xanthineguanine phosphoribosyl transferase, the Herpes simplex virus type 1 thymidine kinase, adenine phosphoribosyltransferase and hypoxanthine phosphoribosyltransferase and/or (b) a negative selectable marker, the marker for example being selected from Herpes simplex virus type 1 thymidine kinase, adenine phosphoribosyltransferase, hygromycin phosphotransferase and hypoxanthine phosphoribosyltransferase.

The use of a positive selectable marker facilitates the selection and/or identification of transfected T lymphocytes, whereas the presence of a negative selectable marker permits the subsequent elimination of the transfected T cells either in vivo or in vitro (for example if undesirable side effects arise).

The methods of plasmid preparation so as to produce constructs to allow the transcription and translation of e.g. the α and β units in mammalian cells, and in particular, in the engineered cytotoxic T-lymphocytes, are well known to the man skilled in the art.

A particularly advantageous vector expresses both the α and β chains under the control of a single promoter. Particularly preferred are promoters specific for T-cell and/or T-cell precursor/progenitor cell expression, and the constructs may be separated by a poliovirus derived internal ribosomal entry site (IRES). The IRES allows two separate genetic elements to be expressed under the control of the same promoter/enhancer element placed at the 5' end of the sequence.

The vector may advantageously be a retroviral vector. Retroviral transduction of mammalian cells is very efficient and can be used to transduce human cells with genes such as the α and β chains of a selected T-cell receptor. The retroviral vector preferably has a specific 3' LTR deletion. Upon transduction with the vector of the cell to be engineered, and the vector's reverse transcription to form proviral DNA for subsequent incorporation into the hosts genome, the 5' LTR promoter/enhancer element activity would be lost. This would allow expression to be placed under the control of selected promoters, thus permitting cytotoxic T-cell-specific expression (Yu SF et al 1986 PNAS USA 83 3194). If vector size constraints are too limited in such a retroviral vector, however, alternative vectors such as adenovral vectors could also be considered.

The targeted cytotoxic T lymphocytes and vectors of the invention find application in various forms of therapy, and in particular in adoptive immunotherapy. The lymphocytes and vectors are particularly useful in adoptive immunotherapy comprising the steps of; (a) removing cytotoxic T-lymphocytes from a patient and optionally selectively expanding them in tissue culture, (b) transfecting the cytotoxic T lymphocytes removed in step (a) with the vector of the invention to produce targeted cytotoxic T lymphocytes, and (c) reintroducing the targeted cytotoxic T lymphocytes of step (b) into the patient.

In the method described above, the T-lymphocytes used in the adoptive immunotherapy are autologous. This has particular advantages, because it ensures that the T-lymphocytes have the appropriate costimulatory and adhesion factors necessary for efficient recognition of target cells after reintroduction into the patient.

The therapy may be applied to cancer patients, AIDS patients, individuals suffering from an autoimmune disease or immunosuppressed individuals (for example individuals bearing a transplanted organ) suffering an opportunistic infection.

The targeted cytotoxic T lymphocytes and vectors of the invention also find application as vaccines, e.g. for prophylactic use in individuals at high risk of disease. Examples of such high risk individuals include individuals predisposed by genetic or environmental factors to disease (e.g. cancer, AIDS or hepatitis). For example, the T-lymphocytes of the present invention could be used to provide immunity against AIDS of the type disclosed by Rowland-Jones et al. (1995), Nature Medecine, Vol. 1 (1), pages 59-64.

The invention will now be described in more detail by way of specific examples of proposed protocols which are believed to be practicable (with or without modification). These examples are not intended to be taken as limiting in any way.

### Example 1: TcR Cloning

Cloning of T cell receptors has historically relied on the formation of cDNA libraries from the T cell carrying the receptor of interest. This has meant that for each T cell receptor a new cDNA library has had to be made from the appropriate clonal cell line, which is a time consuming process.

However, between the genes of the two subunits of the T cell receptor, α and β, there are regions of sequence identity. These are known as the constant regions and are found in every α and β subunit gene. The sequences for these regions can be found from published data of cloned T cell receptor subunits, and by comparing these it is possible to identify constant regions of sequence identity. Although these regions are called constant regions, analysis has shown that there is some variation between constant regions that have so far been cloned. However, small regions of consistent sequences can be identified within a receptor subunit.

Using these regions it is possible to clone the full length α and β subunit transcripts by a method known as inverse polymerase chain reaction (PCR) described by Ochman et al (Genetics (1988) 120: 621-623). This method relies on the ability of cDNA, derived from mRNA, to be circularised and using PCR primers designed against the known sequence of the transcript, amplify the whole cDNA by chain elongation across the uncharacterised region of the circularised DNA. However the resulting PCR products do not give the sequence of interest in the correct orientation, and it is therefore necessary to clone the PCR products to sequence them. Using the sequence data it is possible to visualise the sequence in the correct orientation and design normal PCR primers to amplify the full length cDNA in the correct orientation. These cDNA clones can then be cloned into expression vectors for further manipulation.

Since the inverse primers have been designed against a constant region, the same methodology and primers can be used for different T cell clones. This significantly reduces the cell number requirements and increases the speed and efficiency of TcR cloning.

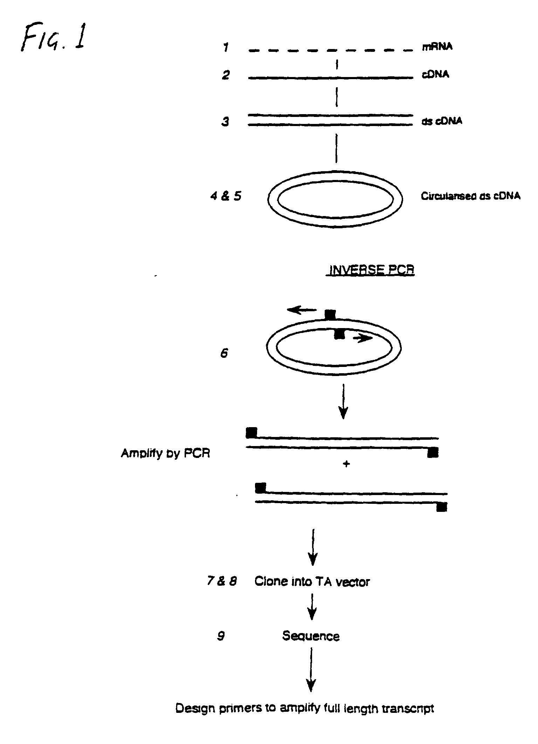

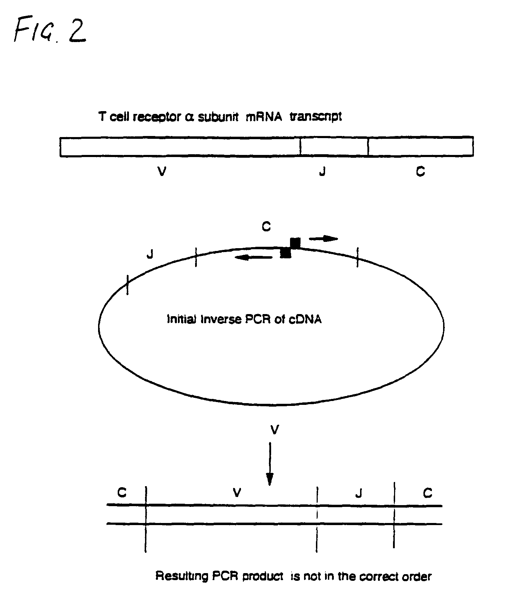

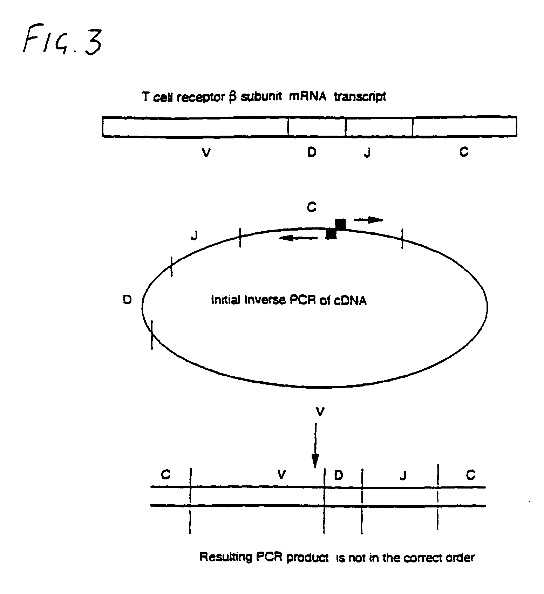

The method can be summarized as follows (see Figures 1 to 3):
1) Total RNA is isolated from the T cell clone of interest using Trizol reagent.
2) cDNA is produced from the total RNA by reverse transcription using an Oligo dT as the primer for transcription.
3) Second strand synthesis is achieved by DNA polymerase 1 in the presence of RNase H to create nicks and gaps in the hybridised mRNA strand, which provides 3'-OH priming sites for DNA synthesis.
4) Circularisation is performed with T4DNA ligase in a dilute DNA concentration that favours the formation of monomeric circles.
5) Linear cDNA is then removed by treatment with exonuclease III.
6) Using the resulting closed circular cDNA, the transcripts of interest are amplified by inverse PCR. The inverse primers are designed in such a way as to initially replicate the cDNA in opposite directions (as shown on Figure 1) giving linear products. These products then replicate as expected with normal PCR methodology.
7) The PCR products are separated on an agarose gel and several of the largest bands are excised from the gel and purified. The purified products are then used in a further PCR reaction. However, as a control, normal PCR primers designed to amplify a short length of the constant region of one of the T cell receptor subunits, are used. As the fragments obtained from the inverse PCR will contain constant regions, if they are truly T cell receptor subunit transcripts, normal PCR using these primers will amplify short fragments which can be visualised on an agarose gel. Fragments which did not give normal PCR products when amplified using the appropriate normal primers do not represent clones of interest and are discarded.
8) The remaining fragments can be cloned into the TA vector (available from Invitrogen) which is a plasmid vector which relies on the 3' adenylation sites created during each round of replication by Taq polymerase, to incorporate the PCR product. Competent bacteria are then transformed with the resulting plasmids, and transformants selected by ampicillin resistance. Plasmids with inserts have a disrupted lac Z gene and therefore appear white when plated on nutrient agar containing X-gal.
9) Confirmatory digests are carried out on mini-preps of plasmid DNA from the selected clones. Selected clones are then expanded and large scale plasmid preps are made for automated sequencing.
10) Once the sequence data has been obtained it is possible to distinguish the correct orientation of the transcript by identifying the start codon ATG (see Figure 2 for more explicit explanation). Using this data it is then possible to design PCR primers to amplify the whole transcript from the original linear cDNA preparation from the T cell clone. These can then be cloned into a variety of vectors, including eukaryotic expression vectors.

It is posible to use this methodology for a limitless number of T cell receptor subunits, providing the appropriate T cell clones are available, without the need for designing new primers (except for the amplification of the full length transcripts at the end of the procedure). In addition the whole procedure can be carried out in a matter of days, as opposed to weeks in the case of cDNA library generation.

### Example 2: Construction of cytotoxic T cells targeted to the MAGE-1 melanoma antigen

Cytotoxic T-lymphocytes recognizing the MAGE-1 gene product MZ2-E (see e.g. Chen et al. (1994), PNAS 91, pp 1004-1008; Van den Eynde et al. (1989), Int. J. Cancer, 44, pp 634-640; van der Bruggen et al. (1991), Science, 254, pp. 1643-1647; Traversari et al. (1992), Immunogenetics, 35, pp. 145-152; Traversari et al. (1992), J. Exp. Med. 176, pp. 1453-1457) were collected and their DNA extracted.

A complementary DNA library was constructed from the extracted DNA using a lambda gt11 vector. Complementary DNA (cDNA) clones encoding the α and β chains were then isolated using α and β chain constant-region hybridization probes described in e.g. Dembic et al. (1985) Nature 314, pp. 271-273 and Snodgrass et al. (1985) Nature 315, pp. 232-233. The procedure used was similar to that described in Dembic et al. (1986) Nature, 320, pp. 232-238.

The genes for both α and β chains were then cloned into a cosmid vector in the same transcriptional orientation, together with the neomycin-resistance gene as a positive selection marker. Appropriate expression elements were functionally coupled to the α and β coding sequences to ensure efficient expression.

The cosmid vector containing the α and β chains was then transferred by protoplast fusion into a suitable recipient cytolytic T cell hybridoma to yield a recombinant T-cell hybridoma having specificity for the MAGE-1 gene product.

Those skilled in the art will realize that the above described method can be readily adapted to the recovery and transfer of other TcRs, and can be applied with appropriate modifications to any recipient cytotoxic T-cell population.

### Example 3: Generation of Human T-lymphocytes genetically engineered to express cytotoxic activity against cells expressing the influenza (Flu) virus.

Human whole peripheral blood mononuclear cells (PBMC), derived from a class 1 MCH HLA-A2 blood donor, were prepared and cultured in-vitro in standard medium in the presence of Flu peptides known to be presented in a HLA-A2 restricted fashion. Thus, antigen presenting cells were formed.

Alternatively, antigen presenting cells can be formed by the addition of peptide to a population of dendritic cells isolated from a HLA-A2 restricted donor.

After 24 hours incubation with peptide, the PBMC's were added to CD4/CD8 cells derived from a recently influenza immunized human donor, also of the HLA-A2 class 1 MHC restricted idiotype. The CD4/CD8 cell population derived from the immunized donor was plated in limiting dilution (100 down to 1 cell) in microtitre plates and left in the presence of the presenting cells for 10-14 days with appropriate medium changes. The plates were then screened for expanding colonies of T-lymphocytes which represented activated CD4/CD8 cells capable of recognizing the antigen presenting cells and being cytotoxic to them.

These expanding colonies of activated T-lymphocytes, capable of recognising influenza antigen when presented by cells in a class 1 MHC restricted fashion, were then cloned and used to prepare complete T-cell receptor α and β chain sequences as described in Example 1.
Alternatively, other methods of producing and isolating the α and β cDNA known to the man skilled in the art can be employed.

Once isolated, the cDNA of the α and β chains were then used to engineer T-cells derived from a non-immune class 1 MHC HLA-A2 donor. This conferred to the genetically engineered T-cells the ability to recognize influenza infected cells in a class 1 restricted fashion and mediate cytotoxicity as determined by chromium release assay.

### Example 4: Generation of human T-lymphocytes genetically engineered to express cytotoxic activity against cells expressing a member of the ras oncogene family.

Human whole peripheral blood mononuclear cells (PMBC), derived from a class 1 MHC HLA-A2 blood donor, were prepared and cultured in-vitro in standard medium in the presence of Harvey ras or Kirsten ras or N-ras oncogene peptides known to be presented in a HLA-A2 restricted fashion. Thus, antigen presenting cells were formed.

Alternatively, antigen presenting cells can be formed by the addition of the said peptide(s) to a population of dendritic cells isolated from a HLA-A2 restricted donor.

After 24 hours incubation with peptide the PBMC's were added to CD4/CDB cells derived from a series of human donors, also of the HLA-A2 class 1 MHC restricted idiotype. CD4/CD8 cell populations derived from the donors were plated in limiting dilution (100 down to 1 cell) in microtite plates and left in the presence of the presenting cells for 10-14 days with appropriate medium changes. The plates were then screened for expanding colonies of T-lymphocytes which represented activated CD4/CD8 cells capable of recognizing the antigen presenting cells and being cytotoxic to them.

These expanding colonies of activated T-lymphocytes, capable of recognising ras oncogene antigen when presented by cells in a class 1 MHC restricted fashion, were then cloned and used to prepare complete T-cell receptor α and β chain sequences as described earlier.

Once isolated, the cDNA of the α and β chains relating to recognition of either the Harvey ras or Kirsten ras of N-ras oncogene peptides was then used to engineer T-cells derived from a non-immune class 1 MHC HLA-A2 donor. This conferred to the genetically engineered T-cells the ability to recognize ras oncogene activation in cells in a class 1 restricted fashion and provided cell mediated cytotoxicity to such cells as determined by chromium release assay.

### Example 5 - Generation of human T-lymphocytes genetically engineered to express cytotoxic activity against cells expressing HIV peptides.

Human whole peripheral blood mononuclear cells (PBMC), derived from a class 1 MHC HLA-B35 blood donor, were prepared and cultured in-vitro in standard medium in the presence of HIV peptides known to be presented in a HLA-B35 restricted fashion. Thus, antigen presenting cells were formed.

Alternatively, antigen presenting cells can be formed by the addition of peptide to a population of dendritic cells isolated from a HLA-B35 restricted donor.

After 24 hours incubation with peptide, the PBMC's were added to CD4/CD8 cells derived from human donor with potential HIV immunity, also of the HLA-B35 class 1 MHC restricted idiotype. The CD4/CD8 cell population derived from the potentially immune donor was plated in limiting dilution (100 down to 1 cell) in microtitre plates and left in the precence of the presenting cells for 10-14 days with appropriate medium changes. The plates were then screened for expanding colonies of T-lymphocytes which represented activated CD4/CD8 cells capable of recognizing the antigen presenting cells and those which were cytotoxic to them.

These expanding colonies of activated T-lymphocytes, capable of recognising HLA-B35 processed HIV antigen when presented by cells in a class 1 MHC restricted fashion, were then cloned and used to prepare complete T-cell receptor α and β chain sequences as described earlier, or by alternative methods of producing and isolating the α and β cDNA known to the man skilled in the art.

Once isolated, the cDNA of the α and β chains was then used to engineer T-cells derived from a non-immune class 1 MHC HLA-B35 donor. This conferred to the genetically engineered T-cells, recognition of HIV infected cells in a class 1 MHC restricted fashion. Cell mediated cytotoxicity by these engineered cells was shown by chromium release assay.

## Claims

1. A targeted cytotoxic T lymphocyte having a TcR comprising heterologous α and β polypeptides, which heterologous polypeptides confer on the lymphocyte MHC Class I-restricted specificity for disease-causing target cells, wherein the T lymphocyte is monovalent, having a single species of TcR conferring MHC Class I-restricted specificity for a single class of target cells, wherein the heterologous TcR polypeptides are dervied from the expression of recombinant or wholly synthetic DNA, or wherein the heterologous TcR polypeptides are derived, directly or indirectly from DNA of a donor cytotoxic lymphocyte.

2. The targeted cytotoxic T lymphocyte of claim 1 which is recombinant.

3. The targeted cytotoxic T lymphocyte of claim 2 which is transduced with a viral vector.

4. The targeted cytotoxic T lymphocyte of any one of the preceding claims wherein the target cells comprise tumour cells, immune cells contributing to an autoimmune response and/or cells infected with a pathogen.

5. The targeted cytotoxic T lymphocyte of claim 4 wherein the target cells comprise human melanoma cells and the heterologous α and β polypeptides confer specificity for; (a) the MAGE-1 tumcur antigen. (b) the MAGE-3 tumour antigen, (c) the MART 1/Aa tumour antigen, (d) the gp100 tumour antigen and/or (e) the tyrosinase tumour antigen.

6. The targeted cytotoxic T lymphocyte of claim 4 wherein the pathogen is a virus.

7. The targeted cytotoxic T lymphocyte of claim 6 wherein the target cells comprise HIV-infected lymphocytes.

8. The targeted cytotoxic T lymphocyte of any one of the preceding claims wherein the heterologcus α and β TcR polypeptides are provided as a single fusion polypeptide.

9. The targeted cytotoxic T lymphocyte of any one of the preceding claims wherein the heterologous α and β TcR polypeptides are chimaeric.

10. The targeted cytotoxic T lymphocyte of claim 9 wherein the chimaeric TcR polypeptides comprise an immunoglobulin variable domain or fragment thereof.

11. A method for producing the targeted cytotoxic T lymphocyte of any one of the preceding claims. the method comprising the steps of:
(a) providing a vector comprising nucleic acid (e.g. DNA derived from a donor cytotoxic T lymphocyte) encoding α and β TcR polypeptides specific for disease-causing target cells, and
(b) transfecting a recipient cytotoxic T lymphocyte with the vector of step (a) to produce a recombinant cytotoxic T lymphocyte having DNA encoding α and β TcR polypeptides specific for disease-causing target cells,
whereby the recombinant cytotoxic T lymphocyte of step (b) expresses the DNA encoding α and β TcR polypeptides to endow the lymphocyte with MHC-Class I restricted specificity and thereby target it to the target cells.

12. The method of claim 11 wherein the vector of step (a) is provided by cloning, assembling or synthesising (e.g. by solid phase oligonucleotide synthesis) DNA encoding α and β TcR polypeptides specific for disease-causing target cells.

13. The method of claim 12 wherein the DNA encoding the α and β TcR polypeptides is cloned by:
(a) obtaining a sample or donor T lymphocytes, e.g. from a blood bank, blood sample or tumour biopsy;
(b) enriching the sample of donor T lymphocytes for cytotoxic T lymphocytes having specificity for disease-causing target cells. e.g. by specifically induced proliferation and/or specific clonal expansion;
(c) extracting chromosomal DNA from the donor cytotoxic T lymphocytes; and
(d) isolating DNA encoding the α and β TcR polypeptides, e.g. by primer-specific PCR amplification.

14. The method of any one of claims 11 to 13 wherein the vector is transfected into the recipient cytotoxic T lymphocyte by electroporation, protoplast fusion or viral (e.g. retroviral) transfection.

15. A targeted cytotoxic T lymphocyte producible by the method of any one of claims 11 to 14.

16. A vector for use in the method of any one of claims 11 to 14 compnsing DNA encoding α and β TcR polypeptides specific for a disease-causing cell, the DNA for example being operably linked to an expression element or elements, the expression element or elements being selected for example from transcriptional and/or translational elements, promoters, ribosome binding sites, enhancers, regulatory sites (e.g. activator and repressor (operator) sites) and expression elements naturally associated with the α and/or β TcR peptide genes.

17. The vector of claim 16 which is a viral vector, being for example based on simian virus 40, adenoviruses (e.g. human adenoviruses), retroviruses, and papillomavirus.

18. The vector of claim 16 or claim 17 further comprising; (a) a positive selectable marker, the marker for example being selected from neomycin phosphotransferase, hygromycin phosphotransferase, xanthineguanine phesphoribosyl transferase. the Herpes simplex virus type 1 thymidine kinase. adenine phosphoribosyltransferase and hypoxanthine phosphonbosyltransferase and/or (b) a negative selectable marker, the marker for example being selected from Herpes simplex virus type 1 thymidine kinase, adenine phosphoribosyltransferase. hygromycin phosphotransferase and hypoxanthine phosphoribosyltransferase.

19. A targeted cyrotoxic T lymphocyte according to any one of claims 1 to 10 or 15 having a TcR comprising heterologous α and β polypeptides. which heterologous polypeptides confer on the lymphocyte MHC Class I-restricted specificity for a target cell, for use in therapy.

20. Use of; (a) the targeted cytotoxic T lymphocyte as defined in claim 19, or (b) the vector of any one of claims 16 to 18, for the preparation of a medicament for use in adoptive immunotherapy.

21. The use of claim 20 wherein the patient is a cancer patient, an AIDS patient, an individual suffering from an autoimmune disease or an immunosuppressed individual (for example an individual bearing a transplanted organ) suffenng an opportunistic infection,

22. Use of: (a) the targeted cytotoxic T lymphocyte as defined in claim 19, or (b) the vector of any one of claims 16 to 18, for the preparation of a vaccine for use in immunotherapy or prophylaxis, e.g. for use in the treatment or prophylaxis of AIDS.

23. A vaccine comprising the targeted cytotoxic T lymphocyte according to any one of claims 1 to 10 or 15, for example further comprising a pharmaceutically acceptable excipient.

## Patentansprüche

1. Targetierter zytotoxischer T-Lymphozyt mit einem TcR, umfassend heterologe α- und β-Polypeptide, wobei die heterologen Polypeptide auf den Lymphozyt auf MHC Klasse I beschränkte Spezifität für krankheitsverursachende Targetzellen übertragen, wobei der T-Lymphozyt monovalent ist, mit einer einzelnen TcR-Gattung, die auf MHC Klasse I beschränkte Spezifität für eine einzelne Klasse von Targetzellen überträgt, wobei die heterologen TcR-Polypeptide von der Expression rekombinanter oder völlig synthetischer DNA abgeleitet sind, oder wobei die heterologen TcR-Polypeptide direkt oder indirekt von der DNA eines zytotoxischen Donor-Lymphozyten abgeleitet sind.

2. Targetierter zytotoxischer T-Lymphozyt nach Anspruch 1, der rekombinant ist.

3. Targetierter zytotoxischer T-Lymphozyt nach Anspruch 2, der mit einem viralen Vektor transduziert ist.

4. Targetierter zytotoxischer T-Lymphozyt nach einem der vorherigen Ansprüche, wobei die Targetzellen Tumorzellen, Immunzellen, die zu einer Autoimmunreaktion beitragen, und/oder mit einem Pathogen infizierte Zellen umfassen.

5. Targetierter zytotoxischer T-Lymphozyt nach Anspruch 4, wobei die Targetzellen humane Melanomzellen umfassen und die heterologen α- und β-Polypeptide Spezifität für (a) das MAGE-1-Tumorantigen, (b) das MAGE-3-Tumorantigen, (c) das MART 1/Aa-Tumorantigen, (d) das gp100-Tumorantigen und/oder (e) das Tyrosinase-Tumorantigen übertragen.

6. Targetierter zytotoxischer T-Lymphozyt nach Anspruch 4, wobei das Pathogen ein Virus ist.

7. Targetierter zytotoxischer T-Lymphozyt nach Anspruch 6, wobei die Targetzellen HIV-infizierte Lymphozyten umfassen.

8. Targetierter zytotoxischer T-Lymphozyt nach einem der vorherigen Ansprüche, wobei die heterologen α- und β-TcR-Polypeptide als einzelnes Fusionspolypeptid bereitgestellt werden.

9. Targetierter zytotoxischer T-Lymphozyt nach einem der vorherigen Ansprüche, wobei die heterologen α- und β-TcR-Polypeptide chimär sind.

10. Targetierter zytotoxischer T-Lymphozyt nach Anspruch 9, wobei die chimären TcR-Polypeptide eine variable Immunglobulindomäne oder ein Fragment davon umfassen.

11. Verfahren zur Herstellung des targetierten zytotoxischen T-Lymphozyten nach einem der vorherigen Ansprüche, wobei das Verfahren die folgenden Schritte umfasst:
(a) Bereitstellen eines Vektors, umfassend Nucleinsäure (z.B. von einem zytotoxischen Donor-T-Lymphozyten abstammende DNA), die α- und β-TcR-Polypeptide kodiert, die für krankheitsverursachende Targetzellen spezifisch sind, und
(b) Transfizieren eines zytotoxischen Rezipienten-T-Lymphozyten mit dem Vektor aus Schritt (a), um einen rekombinanten zytotoxischen T-Lymphozyt mit einer DNA zu produzieren, die α- und β-TcR-polypeptide kodiert, die für krankheitsverursachende Targetzellen spezifisch sind,
wobei der rekombinante zytotoxische T-Lymphozyt aus Schritt (b) die DNA exprimiert, die α- und β-TcR-Polypeptide kodiert, um dem Lymphozyt auf MHC Klasse I beschränkte Spezifität zu verleihen und ihn somit auf die Targetzellen zu targetieren.

12. Verfahren nach Anspruch 11, wobei der Vektor aus Schritt
(a) bereitgestellt wird durch Klonieren, Zusammensetzen oder Synthetisieren (z.B. durch Festphasen-Oligonucleotidsynthese) der DNA, die α- und β-TcR-Polypeptide kodiert, die für krankheitsverursachende Targetzellen spezifisch sind.

13. Verfahren nach Anspruch 12, wobei die DNA, die die α- und β-TcR-Polypeptide kodiert, kloniert wird durch:
(a) Gewinnen einer Probe von Donor-T-Lymphozyten, z.B. von einer Blutbank, Blutprobe oder Tumorbiopsie;
(b) Anreichern der Probe von Donor-T-Lymphozyten mit zytotoxischen T-Lymphozyten mit Spezifität für krankheitsverursachende Targetzellen, z.B. durch speziell induzierte Proliferation und/oder spezifische klonale Expansion;
(c) Extrahieren von chromosomaler DNA von den zytotoxischen Donor-T-Lymphozyten; und
(d) Isolieren von DNA, die die α- und β-TcR-Polypeptide kodiert, z.B. durch Primer-spezifische PCR-Amplifikation.

14. Verfahren nach einem der Ansprüche 11 bis 13, wobei der Vektor in den zytotoxischen Rezipienten-T-Lymphozyt durch Elektroporation, Protoplastenfusion oder virale (z.B. retrovirale) Transfektion transfiziert wird.

15. Targetierter zytotoxischer T-Lymphozyt, der mit dem Verfahren aus einem der Ansprüche 11 bis 14 produzierbar ist.

16. Vektor zur Verwendung in dem Verfahren aus einem der Ansprüche 11 bis 14, umfassend DNA, die α- und β-TcR-Polypeptide kodiert, die für eine krankheitsverursachende Zelle spezifisch sind, wobei die DNA zum Beispiel mit (einem) Expressionselement oder -elementen funktionell verbunden ist, wobei das/die Expressionselement oder -elemente zum Beispiel ausgewählt ist/sind aus Transkriptions- und/oder Translationselementen, Promotoren, Ribosombindungsstellen, Enhancer, Regulationszentren (z.B. Aktivator- und Repressor-(Operator)-Stellen) und Expressionselementen, die mit den α-und/oder β-TcR-Peptidgenen natürlich assoziiert sind.

17. Vektor nach Anspruch 16, der ein viraler Vektor ist und zum Beispiel auf Simian-Virus-40-Adenoviren (z.B. humane Adenoviren), Retroviren und Papillomvirus basiert.

18. Vektor nach Anspruch 16 oder Anspruch 17, ferner umfassend: (a) einen positiven selektierbaren Marker, wobei der Marker beispielsweise ausgewählt ist aus Neomycin-Phosphotransferase, Hygromycin-Phosphotransferase, Xanthinguanin-Phosphoribosyltransferase, der Herpes-simplex-Virus Typ 1 Thymidinkinase, Adenin-Phosphoribosyltransferase und Hypoxanthin-Phosphoribosyltransferase und/oder (b) einen negativen selektierbaren Marker, wobei der Marker beispielsweise ausgewählt ist aus Herpes-simplex-Virus Typ 1 Thymidinkinase, Adenin-Phosphoribosyltransferase, Hygromycin-Phosphotransferase und Hypoxanthin-Phosphoribosyltransferase.

19. Targetierter zytotoxischer T-Lymphozyt nach einem der Ansprüche 1 bis 10 oder 15, mit einem TcR, umfassend heterologe α- und β-Polypeptide, wobei die heterologen Polypeptide auf den Lymphozyt auf MHC Klasse I beschränkte Spezifizität für eine Targetzelle übertragen, zur Verwendung in der Therapie.

20. Verwendung von: (a) dem targetierten zytotoxischen T-Lymphozyt nach Anspruch 19, oder (b) dem Vektor aus einem der Ansprüche 16 bis 18, zur Herstellung eines Medikamentes zur Verwendung in der adoptiven Immuntherapie.

21. Verwendung nach Anspruch 20, wobei der Patient ein Krebspatient, ein AIDS-Patient, eine an einer Autoimmunkrankheit leidende Person oder eine immunosupprimierte Person ist (z.B. eine Person mit einem Organtransplantat), die an einer opportunistischen Infektion leidet.

22. Verwendung von: (a) dem targetierten zytotoxischen T-Lymphozyt nach Anspruch 19, oder (b) dem Vektor nach einem der Ansprüche 16 bis 18, zur Herstellung eines Impfstoffs zur Verwendung in der Immuntherapie oder Prophylaxe, z.B. zur Verwendung bei der Behandlung oder Prophylaxe von AIDS.

23. Impfstoff, umfassend den targetierten zytotoxischen T-Lymphozyt nach einem der Ansprüche 1 bis 10 oder 15, beispielsweise ferner umfassend ein pharmazeutisch akzeptables Bindemittel.

## Revendications

1. Lymphocyte T cytotoxique ciblé ayant un TcR comprenant des polypeptides hétérologues α et β, lesquels polypeptides hétérologues confèrent au lymphocyte une spécificité restreinte au MHC Classe I pour des cellules cibles causant une maladie, où le lymphocyte T est monovalent, ayant une seule espèce de TcR conférant une spécificité restreinte au MHC Classe I pour une seule classe de cellules cibles, où les polypeptides TcR hétérologues sont dérivés de l'expression d'un ADN recombiné ou entièrement synthétique, ou bien où les polypeptides TcR hétérologues sont dérivés, directement ou indirectement, de l'ADN d'un lymphocyte cytotoxique d'un donneur.

2. Le lymphocyte T cytotoxique ciblé de la revendication 1 qui est recombiné.

3. Le lymphoyte T cytotoxique ciblé de la revendication 2 qui est transduit d'un vecteur viral.

4. Le lymphocyte T cytotoxique ciblé de l'une quelconque des revendications précédentes où les cellules cibles comprennent des cellules tumorales, des cellules immunes contribuant à une réponse auto-immune et/ou des cellules infectées avec un pathogène.

5. Le lymphocyte T cytotoxique ciblé de la revendication 4 où les cellules cibles comprennent des cellules de mélanomes humains et les polypeptides hétérologues α et β confèrent une spécificité pour (a) l'antigène tumoral MAGE-1, (b) l'antigène tumoral MAGE-3, (c) l'antigène tumoral MART 1/Aa, (d) l'antigène tumoral gp 100 et/ou (e) l'antigène tumoral tyrosinase.

6. Le lymphocyte T cytotoxique ciblé de la revendication 4 où le pathogène est un virus.

7. Le lymphocyte T cytotoxique ciblé de la revendication 6 où les cellules cibles comprennent des lymphocytes infectés par le VIH.

8. Le lymphocyte T cytotoxique ciblé de l'une quelconque des revendications précédentes où les polypeptides hétérologues α et β sont fournis comme un seul polypeptide de fusion.

9. Le lymphocyte T cytotoxique ciblé de l'une quelconque des revendications précédentes où les polypeptides hétérologues α et β sont chimériques.

10. Le lymphocyte T cytotoxique ciblé de la revendication 9 où les polypeptides TcR chimériques comprennent un domaine variable d'immunoglobulines ou fragment de celui-ci.

11. Méthode pour produire le lymphocyte T cytotoxique ciblé de l'une quelconque des revendications précédentes, la méthode comprenant les étapes consistant à :
(a) fournir un vecteur comprenant de l'acide nucléique (par ex. ADN dérivé d'un lymphocyte T cytotoxique d'un donneur) codant les polypeptides TcR α et β spécifiques pour les cellules cibles causant une maladie, et
(b) transfecter un lymphocyte T cytotoxique d'un receveur avec le vecteur de l'étape (a) pour produire un lymphocyte T cytotoxique recombiné ayant un ADN codant les polypeptides TcR α et β spécifiques pour les cellules cibles causant une maladie,
en vertu de quoi le lymphocyte T cytotoxique recombiné de l'étape (b) exprime l'ADN codant les polypeptides TcR α et β afin de donner au lymphocyte une spécificité restreinte au MCH-Classe I et le cibler ainsi sur les cellules cibles.

12. La méthode de la revendication 11, dans laquelle le vecteur de l'étape (a) est fourni en clonant, en assemblant ou en synthétisant (par ex. synthèse oligonucléotidique en phase solide) l'ADN codant les polypeptides TcR α et β spécifiques pour les cellules cibles causant une maladie.

13. La méthode de la revendication 12 dans laquelle l'ADN codant les polypeptides TcR α et β, est cloné :
(a) en obtenant un échantillon de lymphocytes T d'un donneur, par ex d'une banque de sang, d'un échantillon de sang ou d'une biopsie de tumeur;
(b) en enrichissant l'échantillon de lymphocytes T d'un donneur pour les lymphocytes T cytotoxiques ayant une spécificité pour les cellules cibles causant une maladie, par ex. par prolifération spécifiquement induite et/ou expansion clonale spécifique;
(c) en extrayant l'ADN chromosomique des lymphocytes T cytotoxiques d'un donneur; et
(d) en isolant l'ADN codant les polypeptides TcR α et β, par ex. par amplification de la PCR spécifique à l'amorce.

14. La méthode de l'une quelconque des revendications 11 à 13, dans laquelle le vecteur est transfecté dans le lymphocyte T cytotoxique d'un receveur par électroporation, fusion des protoplastes ou transfection virale (par ex. rétrovirale).

15. Lymphocyte T cytotoxique ciblé productible par la méthode de l'une quelconque des revendications 11 à 14.

16. Vecteur à utiliser dans la méthode de l'une quelconque des revendications 11 à 14, comprenant un ADN codant les polypeptides TcR α et β spécifiques pour une cellule causant une maladie, l'ADN étant, par exemple, lié de manière fonctionnelle à un élément ou des éléments d'expression, l'élément ou les éléments d'expression étant sélectionnés, par exemple, parmi des éléments de transcription et/ou de traduction, des promoteurs, des sites de liaisons ribosomiques, des stimulateurs, des sites régulateurs (par ex. sites activateurs et répresseurs (opérateurs)) et des éléments d'expression naturellement associés aux gènes peptidiques TcR α et/ou β.

17. Le vecteur de la revendication 16 qui est un vecteur viral, étant, par exemple, basé sur le virus simien 40, les adénovirus (par ex. les adénovirus humains), les rétrovirus et le papillomavirus.

18. Le vecteur de la revendication 16 ou de la revendication 17 comprenant en outre :
(a) un marqueur positif sélectionnable, le marqueur étant, par exemple, sélectionné parmi la néomycine phosphotransférase, l'hygromycine phosphotransférase, la xanthineguanine phosphoribosyltransférase, la thymidine kinase du virus de l'herpès simplex de type 1, l'adénine phosphoribosyltransférase et l'hypoxanthine phosphoribosyltransférase et/ou (b) un marqueur négatif sélectionnable, le marqueur étant, par exemple, sélectionné parmi la thymidine kinase du virus de l'herpès simplex de type 1, l'adénine phosphoribosyltransférase, l'hygromycine phosphotransférase et l'hypoxanthine phosphoribosyltransférase.

19. Lymphocyte T cytotoxique ciblé selon l'une quelconque des revendications 1 à 10 ou 15, ayant un TcR comprenant les polypeptides hétérologues α et β, lesquels polypeptides hétérologues confèrent au lymphocyte une spécificité restreinte au MCH Classe 1 pour une cellule cible, à utiliser en thérapie.

20. Utilisation : (a) du lymphocyte T cytotoxique ciblé tel que défini dans la revendication 19, ou (b) du vecteur de l'une quelconque des revendications 16 à 18, pour la préparation d'un médicament à utiliser en immunothérapie adoptive.

21. L'utilisation de la revendication 20, dans laquelle le patient est un patient cancéreux, un patient atteint du SIDA, un individu souffrant d'une maladie auto-immune ou un individu immunodéprimé (par exemple in individu porteur d'un organe transplanté) souffrant d'une infection opportuniste.

22. Utilisation : (a) du lymphocyte T cytotoxique ciblé tel que défini dans la revendication 19, ou (b) du vecteur de l'une quelconque des revendications 16 à 18, pour la préparation d'un vaccin à utiliser en immunothérapie ou en prophylaxie, par exemple à utiliser dans le traitement ou la prophylaxie du SIDA.

23. Vaccin comprenant le lymphocyte T cytotoxique ciblé selon l'une quelconque des revendications 1 à 10 ou 15, comprenant, par exemple, en plus un excipient pharmaceutiquement acceptable.
